# EUROPÄISCHE PATENTSCHRIFT

(11) **EP 1 477 992 B1**
(45) Veröffentlichungstag und Bekanntmachung des Hinweises auf die Patenterteilung: **30.12.2009**
(21) Anmeldenummer: 04007089.8
(22) Anmeldetag: 24.03.2004
(51) Int. Cl.: G21F 3/00, A61B 6/10

(54) **Strahlenschutzvorrichtung mit transparenter Strahlenschutzscheibe**
Radiation shielding device with transparent radiation shielding window pane
Dispositif de protection contre les radiations avec vitre faisant écran

(30) Priorität: 15.05.2003 DE 20307606 U
(43) Veröffentlichungstag der Anmeldung: 17.11.2004
(73) Patentinhaber: Mavig GmbH, 81829 München (DE)
(72) Erfinder: Ballsieper, Barbara, 82024 Taufkirchen (DE)
(74) Vertreter: Körfer, Thomas

(56) Entgegenhaltungen:
- EP-A- 0 345 548
- DE-A- 4 301 908
- DE-A- 19 643 623
- US-A- 4 023 219
- US-A- 4 581 538

## Beschreibung

Die Erfindung betrifft eine Strahlenschutzvorrichtung, insbesondere eine Strahlenschutzvorrichtung zur Abschirmung der durch eine Röntgenstrahlungsquelle abgestrahlte Röntgenstrahlung, mit einer transparenten Strahlenschutzscheibe gemäß Anspruch 1.

Aus der DE 196 43 623 A1 ist ein Strahlenschutzstuhl bekannt, bei welchem eine durchsichtige, strahlenabsorbierende Scheibe und eine Steh-Sitzhilfe zu einer Einheit verknüpft sind. Die Scheibe ist höhenverstellbar und weist Einbuchtungen als Durchgriffe auf. Der Strahlenschutzstuhl ist mit Rädern versehen und kann dadurch relativ zum Untersuchungsfeld verschoben werden.

Nachteilig bei der aus der DE 196 43 623 A1 bekannten Strahlenschutzvorrichtung ist insbesondere, daß die den Strahlenschutzstuhl benutzende Person, beispielsweise ein Arzt, ein Assistent oder eine Krankenschwester, sich bedingt durch die sitzende Position in einer verhältnismäßig großen Entfernung zu der schützenden Scheibe befindet. Dadurch ist einerseits die Bewegungsfreiheit der Person stark eingeschränkt, da die oberen Extremitäten nicht weit genug über die Scheibenebene hinausgestreckt werden können. Andererseits ist die Gefahr einer Strahlenexposition durch die große Entfernung zur Scheibe u. U. erhöht, da sich die Person, um an Geräte, Bedienschalter etc. herankommen zu können, unbeabsichtigt aus dem Schutz der Scheibe hinausbeugen und sich dadurch der Strahlung aussetzen könnte.

Weiterhin ist der große und unhandliche Strahlenschutzstuhl schwer zu versetzen und trotz Rollen nur unter einem erheblichen Kraftaufwand verschiebbar. Dies ist bei Operationsbetrieb entschieden von Nachteil, da die Bewegungsfreiheit und der Aktionsradius der den Strahlenschutzstuhl , benutzenden Person erheblich eingeschränkt ist.

Aufgabe der Erfindung ist es daher, eine Strahlenschutzvorrichtung zu schaffen, die den erforderlichen Schutz vor Strahlung, insbesondere vor Röntgenstrahlung, gewährleistet, ausreichende Bewegungsfreiheit bietet und gleichzeitig in einfacher Weise für die Anwendung im operativen Bereich mitgeführt und beliebig angeordnet werden kann.

Die Aufgabe wird durch eine erfindungsgemäße Strahlenschutzvorrichtung mit den Merkmalen des Anspruchs 1 gelöst. Vorteilhafterweise ist die Strahlenschutzvorrichtung mit einer transparenten Strahlenschutzscheibe z. B. aus Bleiacryl versehen, welche an einer Standvorrichtung fixiert ist. Die Strahlenschutzscheibe ist gewölbt ausgeführt und in stehender Körperhaltung des Benutzers körpernah führbar.

Durch die in den Unteransprüchen aufgeführten Maßnahmen sind vorteilhafte Weiterbildungen der im Anspruch 1 angegebenen Strahlenschutzvorrichtung möglich.

Vorteilhaft ist es, daß die Strahlenschutzscheibe in einfacher Weise an zumindest einer, vorzugsweise zwei, Leisten fixiert ist, welche an einem doppelkreuzförmigen Fuß fixiert sind. Die Leisten erstrecken sich entweder mittig oder entlang von Außenkanten der Strahlenschutzscheibe parallel zu dieser und sind beispielsweise mittels Schrauben oder ähnlicher Vorrichtungen mit dieser verbunden.

Weiterhin ist von Vorteil, daß die Arme des Fußes unterschiedlich lang ausgebildet sein können, um das Kipprisiko der Strahlenschutzvorrichtung zu vermindern.

An den Armen angebrachte Rollen ermöglichen eine einfache und schnelle Verschiebung der Strahlenschutzvorrichtung mit geringem Kraftaufwand.

Weiterhin ist von Vorteil, daß die Strahlenschutzvorrichtung mit einer Höhenverstellung versehen sein kann. Diese kann mittels Gasdruckfedern oder auch mechanisch, hydraulisch oder elektrisch betätigbar sein.

Vorteilhafterweise können vorzugsweise asymmetrische Ausnehmungen in der Strahlenschutzscheibe vorgesehen sein, welche das Durchgreifen der Strahlenschutzscheibe ermöglichen und damit einen größeren Aktionsradius und mehr Bewegungsfreiheit ermöglichen.

Von Vorteil ist ebenso, daß die Ausnehmungen bei Bedarf durch Bleilappen abgedeckt werden können, die in geeigneter Weise, wie z. B. mittels Leisten, Druckknöpfen oder Klettbändern, an der Strahlenschutzscheibe fixiert werden können.

Eine Bleischürze am unteren Ende der Strahlenschutzscheibe, welche aus mehreren Schürzenteilen bestehen kann, kann vorteilhafterweise die unteren Extremitäten des Benutzers bis nahe zum Boden schützen. Auch diese kann mittels Leisten, Druckknöpfen oder Klettbändern an der Strahlenschutzscheibe fixiert sein.

Weiterhin ist die Anbringung von abklappbaren Handgriffen an der Strahlenschutzscheibe von Vorteil, wodurch die Verschiebung der Strahlenschutzvorrichtung einfach und kippsicher durchgeführt werden kann und keine Hand- und Fingerabdrücke an der Strahlenschutzscheibe hinterlassen werden.

Auch die Verwendung eines mit dem Fuß verbundenen Sockels zur Führung der Strahlenschutzscheibe ist von Vorteil, da dadurch einerseits die Strahlenschutzscheibe vor Beschädigungen geschützt werden kann, die Verwendung von Strahlenschutzmaterial den Schutz der unteren Extremitäten des Benutzers ermöglicht und eine beliebige Höhenverstellung in einfacher Weise in den Sockel integriert werden kann.

Ausführungsbeispiele der Erfindung sind in den nachfolgenden Zeichnungen vereinfacht dargestellt und in der nachfolgenden Beschreibung näher erläutert. Es zeigen:
- Fig. 1: ein erstes Ausführungsbeispiel einer erfindungsgemäßen Strahlenschutzvorrichtung;
- Fig. 2: ein zweites Ausführungsbeispiel einer erfindungsgemäßen Strahlenschutzvorrichtung;
- Fig. 3: ein drittes Ausführungsbeispiel einer erfindungsgemäßen Strahlenschutzvorrichtung;
- Fig. 4: ein viertes Ausführungsbeispiel einer erfindungsgemäßen Strahlenschutzvorrichtung; und
- Fig. 5: ein fünftes Ausführungsbeispiel einer erfindungsgemäßen Strahlenschutzvorrichtung.

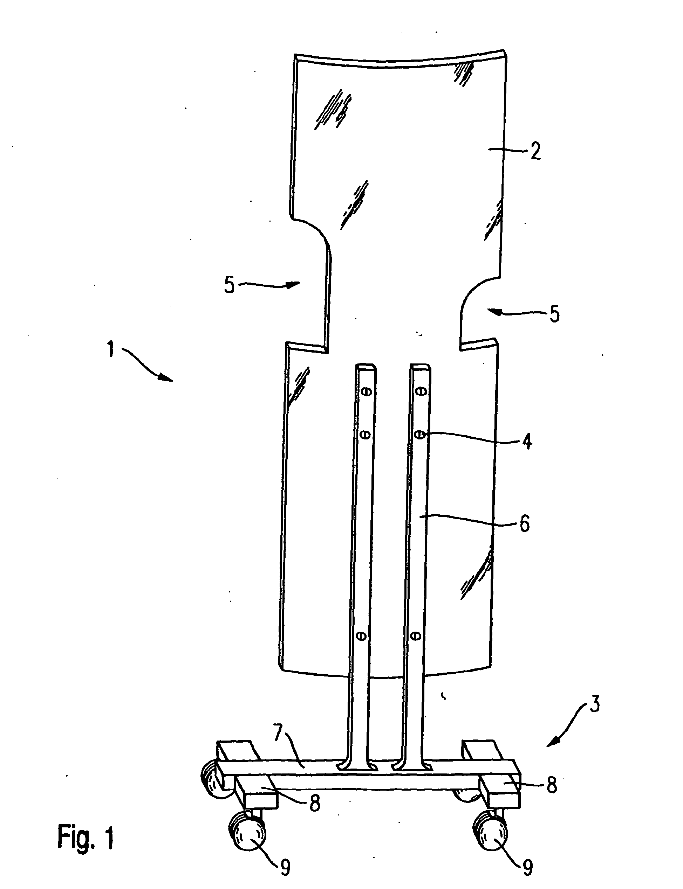

Fig. 1 zeigt in einer schematischen, perspektivischen Darstellung ein erstes Ausführungsbeispiel einer erfindungsgemäßen Strahlenschutzvorrichtung 1. Die erfindungsgemäße Strahlenschutzvorrichtung 1 eignet sich zur Abschirmung einer insbesondere von einer Röntgenquelle abgestrahlten Strahlung, wie sie z.B. im medizinischen Bereich, insbesondere in der radiologisch gestützten Endoskopie, Verwendung findet. Die erfindungsgemäße Strahlenschutzvorrichtung 1 eignet sich jedoch auch für andere Anwendungsfälle.

Die im vorliegenden ersten Ausführungsbeispiel dargestellte Strahlenschutzvorrichtung 1 umfaßt eine Strahlenschutzscheibe 2, welche vorzugsweise aus einem mit Blei legierten Acrylglas besteht, und eine Standvorrichtung 3, an welcher die Strahlenschutzscheibe 2 montiert ist.

Die Strahlenschutzscheibe 2 kann dabei, wie im vorliegenden ersten Ausführungsbeispiel gezeigt, fest beispielsweise mittels Schrauben 4 mit der Standvorrichtung 3 verbunden sein.

Die Strahlenschutzscheibe 2 ist vorzugsweise gewölbt ausgebildet, um der die Strahlenschutzvorrichtung 1 benutzenden Person optimalen Schutz vor der Strahlung zu gewährleisten. Die Strahlenschutzscheibe 2 weist dabei einen Krümmungsradius auf, welcher demjenigen eines normal dimensionierten menschlichen Körpers entspricht. Dadurch kann die die Strahlenschutzvorrichtung 1 benutzende Person sehr nahe an die Strahlenschutzvorrichtung 1 herantreten und verfügt über einen optimalen Aktionsradius.

In der Strahlenschutzscheibe 2 sind Ausnehmungen 5 vorgesehen, welche als Durchgriffe dienen. Diese sind so angeordnet, daß sie sich etwa in Höhe der Ellenbogengelenke der die Strahlenschutzvorrichtung 1 benutzenden Person befinden, so daß die Unterarme durch die Ausnehmungen 5 Zugriff auf Instrumente, Bedienhebel u. ä. erhalten, ohne daß die die Strahlenschutzvorrichtung 1 benutzende Person um die Strahlenschutzscheibe 2 herumgreifen und dadurch mehr Körperfläche der Strahlung aussetzen muß.

Die Ausnehmungen 5 können dabei, wie im Ausführungsbeispiel dargestellt, unterschiedlich groß dimensioniert sein. In Fig. 1 ist die von der Person aus rechte Ausnehmung 5 größer als die linke, da die Mehrzahl der Benutzer Rechtshänder ist und daher mehr mit der rechten Hand agiert.

Die Standvorrichtung 3 weist im wesentlichen zumindest eine, im Ausführungsbeispiel zwei parallele Leisten 6 auf, welche mittels der Schrauben 4 mit der Strahlenschutzscheibe 2 verbunden sind. Die Leisten 6 stehen rechtwinklig mit einem Fuß 7 in Verbindung, welcher vorzugsweise in Form eines Doppelkreuzes ausgebildet ist. Die Arme 8 des Fußes 7 können dabei, wie in Fig. 1 dargestellt, unterschiedlich lang sein, um die Standfestigkeit der Strahlenschutzvorrichtung 1 zu erhöhen und das Kipprisiko zu verringern.

Vorzugsweise ist dabei ein von der die Strahlenschutzvorrichtung 1 benutzenden Person abgewandter Arm 8 verlängert, um das Umkippen nach vorne, von der Person weg, zu verhindern. Ein seitliches Kippen ist aufgrund der Länge des Fußes 7 nahezu unmöglich, und eine Kippbewegung in Richtung auf die Person ist leicht abzufangen. Daher kann die Länge der der Person zugewandten Arme 8 relativ kurz sein, wodurch das Risiko, beim seitlichen Heraustreten beim Verlassen der Strahlenschutzvorrichtung 1 zu stolpern, minimiert wird.

Die Standvorrichtung 3 weist weiterhin an den Armen 8 endständig Rollen 9 auf, welche beweglich sind und eine einfache Verschiebung der Strahlenschutzvorrichtung 1 in jede beliebige Richtung ermöglicht. Die Rollen 9 können auch Bremsen aufweisen, um die Strahlenschutzvorrichtung 1 an ihrem Standort vor unerwünschten Verschiebungen zu sichern.

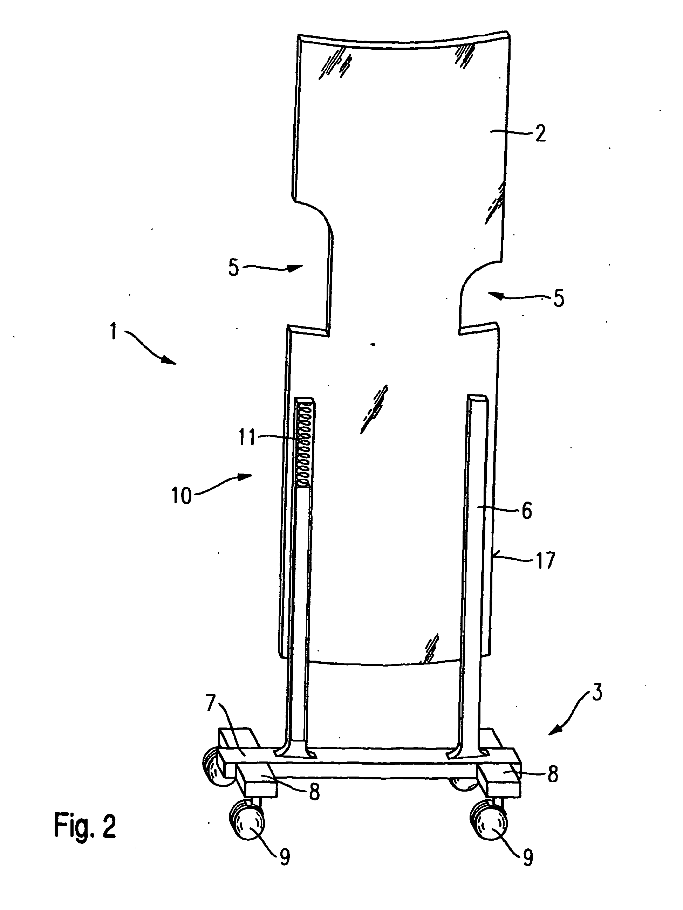

Fig. 2 zeigt in gleicher Darstellung wie Fig. 1 ein zweites Ausführungsbeispiel einer erfindungsgemäß ausgestalteten Strahlenschutzvorrichtung 1. Im Gegensatz zu dem in Fig. 1 dargestellten Ausführungsbeispiel sind hier die die Strahlenschutzscheibe 2 stützenden Leisten 6 möglichst nahe an Außenkanten 17 der Strahlenschutzscheibe 2 angebracht. Dies hat den Vorteil, daß die Strahlenschutzvorrichtung 1 noch näher an eine Patientenliege herangeschoben werden kann, so daß der der die Strahlenschutzvorrichtung 1 benutzenden Person zur Verfügung stehende Aktionsradius vollständig ausgenutzt werden kann. Weiterhin wird der Sichtkontakt auch zu weiter unten befindlichen Gegenständen wie Bedienhebeln nicht eingeschränkt.

weiterhin ist die in Fig. 2 dargestellte Strahlenschutzvorrichtung 1 mit einer Höhenverstellung 10 ausgerüstet. Im Ausführungsbeispiel ist dies durch in den Leisten 6 angeordnete Gasdruckfedern 11 angedeutet, welche eine einfache und zuverlässige Verstellung mit geringem Kraftaufwand ermöglichen.

Andere Möglichkeiten der Höhenverstellung, wie z. B. eine hydraulische Verstellung, mechanische Verstellung mittels eines Handrades, welches in eine Zahnstange eingreift, oder mittels eines elektrischen Antriebs, sind ebenfalls denkbar und in einfacher Weise auf die hier dargestellten Ausführungsbeispiele anwendbar.

Die Länge der Strahlenschutzscheibe 2 ist dabei so zu wählen, daß eine Person mittlerer Größe die Höhe der Strahlenschutzscheibe 2 so wählen kann, daß der gesamte Körper einschließlich der unteren Extremitäten durch die Strahlenschutzscheibe 2 geschützt ist. In Fig. 2 ist die Strahlenschutzscheibe 2 bereits ein Stück nach oben geschoben, so daß die Strahlenschutzvorrichtung 1 auch durch außergewöhnlich große Personen verwendet werden kann.

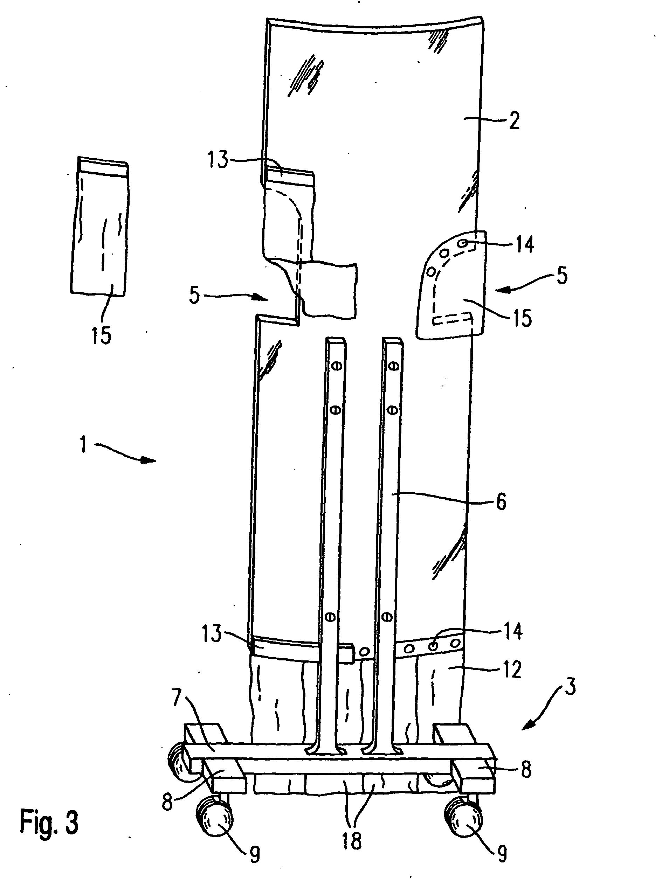

Um in diesem Fall die unteren Extremitäten der die Strahlenschutzvorrichtung 1 benutzenden Person zu schützen, kann eine Bleischürze 12, wie in Fig. 3 dargestellt, verwendet werden. Die Bleischürze 12 kann dabei mittels einer Schiene 13, wie in Fig. 3 links dargestellt, oder mittels Druckknöpfen 14, wie in Fig. 3 rechts dargestellt, an der Strahlenschutzscheibe 2 fixiert sein. In einem nicht sterilen Umfeld können auch Klettbänder oder ähnliches zur Fixierung eingesetzt werden. Zur leichteren Befestigung und zum flexiblen Einsatz mit der Möglichkeit des Durchtretens durch die Bleischürze 12 kann diese aus mehreren einzelnen, einander überlappenden Schürzenteilen 18 bestehen.

In Fig. 3 sind weiterhin Bleilappen 15 dargestellt, welche zur Abdeckung der Ausnehmungen 5 dienen können. Dies ist z. B. dann sinnvoll, wenn die die Strahlenschutzvorrichtung 1 benutzende Person keine aktiven Aufgaben zu erfüllen hat, sondern beispielsweise einer Operation nur als Beobachter beiwohnt. Die Bleilappen 15 decken dabei die Ausnehmungen 15 überlappend ab, um den geltenden Strahlenschutzbestimmungen Genüge zu tun.

Die Bleilappen 15 können ebenfalls mittels Leisten 13 oder Druckknöpfen 14 an der Strahlenschutzscheibe 2 fixiert sein und sind bei Bedarf schnell und einfach abnehmbar. Dies hat den Vorteil, daß die Sterilität durch einfache Reinigung gewährleistet werden kann. In einem nicht sterilen Umfeld können auch Klettbänder oder ähnliches zur Fixierung eingesetzt werden.

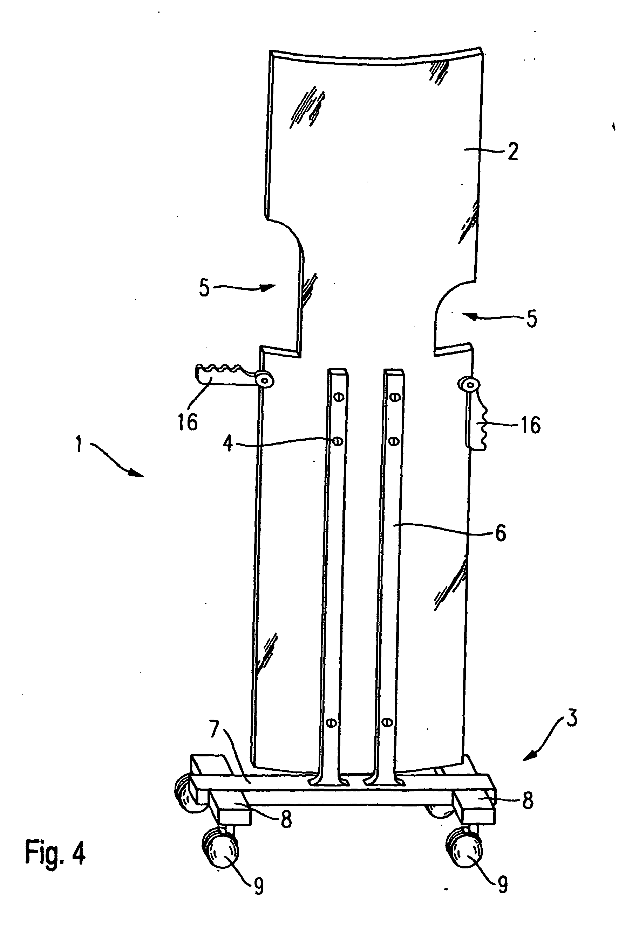

In Fig. 4 ist ein viertes Ausführungsbeispiel einer erfindungsgemäß ausgestalteten Strahlenschutzvorrichtung 1 dargestellt. Hierbei ist die Strahlenschutzscheibe 2 so lang, daß der gesamte Körper der die Strahlenschutzvorrichtung 1 benutzenden Person einschließlich der unteren Extremitäten vor Strahlung geschützt ist. In diesem Fall wird keine Höhenverstellung 10 benötigt.

Um eine einfache Handhabung beim Verschieben der Strahlenschutzvorrichtung 1 zu ermöglichen, weist das in Fig. 4 dargestellte Ausführungsbeispiel seitliche Handgriffe 16 etwa in Ellenbogenhöhe auf. Durch die Handgriffe 16 wird einerseits verhindert, daß die Strahlenschutzscheibe 2 durch seitliches Anfassen beim Verschieben verschmutzt wird, so daß häufiges Abwischen von Hand- und Fingerabdrücken entfallen kann, andererseits visualisiert der Benutzer sofort die korrekte Höhe der Handposition beim Verschieben der Strahlenschutzvorrichtung 1, so daß die Gefahr des Umkippens der Strahlenschutzvorrichtung 1 vermindert wird.

Die Handgriffe 16 können, wie in Fig. 4 rechts dargestellt, heruntergeklappt werden, so daß sie bei Nichtgebrauch nicht behindernd vorstehen.

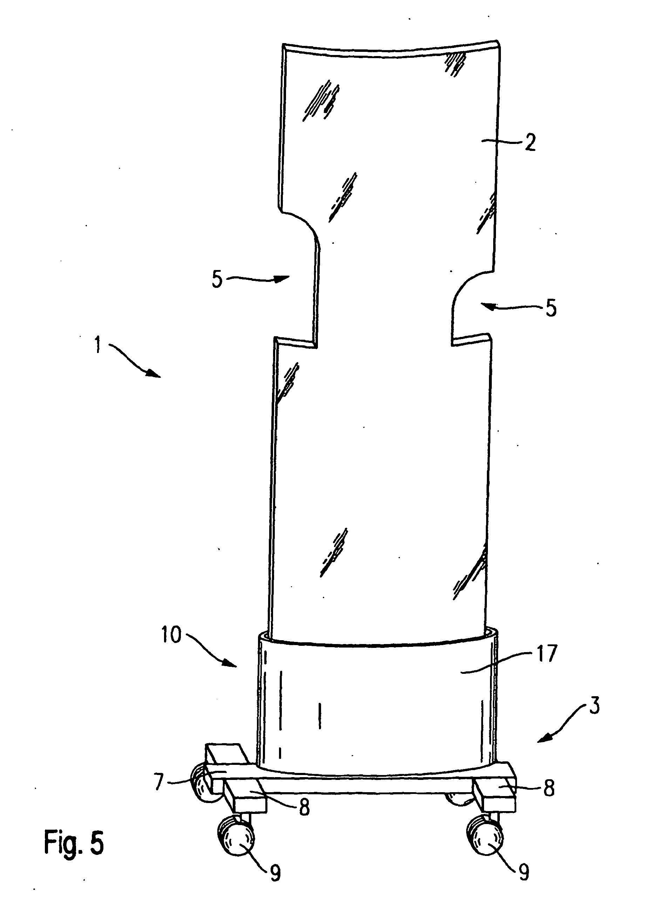

Fig. 5 zeigt ein weiteres vorteilhaftes Ausführungsbeispiel einer erfindungsgemäßen Strahlenschutzvorrichtung 1.

Da die Strahlenschutzvorrichtung 1 auch im Bereich von Diagnosegeräten mit sogenannten C-Röhren Verwendung finden kann, welche sich unter der den Patienten aufnehmenden Untersuchungsliege erstreckt, kann es vorteilhaft sein, die Strahlenschutzscheibe 2 vor Beschädigungen und Kratzern durch einen Sockel 17 zu schützen. Der Sockel 17 weist dabei den gleichen Krümmungsradius wie die Strahlenschutzscheibe 2 auf und ist mit dem doppelkreuzförmigen Fuß 7 verbunden. Dieser kann dabei wie in den vorausgegangenen Ausführungsbeispielen gerade ausgebildet sein, er kann jedoch auch unter einem ähnlichen Krümmungsradius gekrümmt sein wie die Strahlenschutzscheibe 2.

Der Sockel 17 erfüllt durch eine geeignete Materialwahl dann eine mehrfache Aufgabe. Zum einen wird die Strahlenschutzscheibe 2 vor Beschädigungen geschützt, zum anderen kann eine beliebige Höhenverstellung 10 in dem Sockel 17 untergebracht sein, z. B. die weiter oben schon erwähnten Gasdruckfedern 11. Die Strahlenschutzscheibe 2 wird dabei in dem hohlen Sockel 17 geführt und stabilisiert.

Weiterhin können durch die Integration von geeigneten Strahlenschutzmaterialien in wie z. B. Bleiplatten in den Sockel 17 die unteren Extremitäten des Benutzers der Strahlenschutzvorrichtung 1 vor Strahlung geschützt werden, ohne auf eine zusätzliche Vorrichtung wie beispielsweise die Bleischürze 12 gemäß Fig. 3 zurückgreifen zu müssen.

## Patentansprüche

1. Strahlenschutzvorrichtung (1) zur Abschirmung einer von einer
Strahlungsquelle, insbesondere einer Röntgenquelle, abgestrahlten Strahlung, wobei die Strahlenschutzvorrichtung (1) eine die Strahlung absorbierenden, transparenten Strahlenschutzscheibe (2) umfasst, welche an einer Standvorrichtung (3) fixiert ist,
**dadurch gekennzeichnet,**
**dass** die Strahlenschutzscheibe (2) gewölbt ausgeführt ist und in stehender Körperhaltung eines Bedienenden körpernah führbar ist,
wobei die Strahlenschutzscheibe (2) höhenverstellbar an der Standvorrichtung (3) fixiert ist, so dass die unteren Extremitäten des Bedienenden jederzeit vor der Strahlung abgeschirmt sind.

2. Strahlenschutzvorrichtung nach Anspruch 1,
**dadurch gekennzeichnet,**
**dass** die Standvorrichtung (3) zumindest eine Leiste (6) umfasst.

3. Strahlenschutzvorrichtung nach Anspruch 2,
**dadurch gekennzeichnet,**
**dass** die Strahlenschutzscheibe (2) an der zumindest einen Leiste (6) fixiert ist.

4. Strahlenschutzvorrichtung nach Anspruch 3,
**dadurch gekennzeichnet,**
**dass** die Fixierung mittels Schrauben (4), Bolzen oder ähnlicher Befestigungsvorrichtungen erfolgt.

5. Strahlenschutzvorrichtung nach einem der Ansprüche 2 bis 4,
**dadurch gekennzeichnet,**
**dass** die zumindest eine Leiste (6) an der Standvorrichtung (3) und der Strahlenschutzscheibe (2) fixiert ist und sich parallel zu letzterer erstreckt.

6. Strahlenschutzvorrichtung nach Anspruch 1,
**dadurch gekennzeichnet,**
**dass** zwei Leisten (6) vorgesehen sind.

7. Strahlenschutzvorrichtung nach Anspruch 6,
**dadurch gekennzeichnet,**
**dass** sich die zwei Leisten (6) entlang Außenkanten (17) der Strahlenschutzscheibe (2) erstrecken.

8. Strahlenschutzvorrichtung nach einem der Ansprüche 1 bis 7,
**dadurch gekennzeichnet,**
**dass** die Standvorrichtung (3) einen doppelkreuzförmigen Fuß (7) aufweist.

9. Strahlenschutzvorrichtung nach Anspruch 8,
**dadurch gekennzeichnet,**
**dass** der Fuß (7) vier Arme (8) aufweist.

10. Strahlenschutzvorrichtung nach Anspruch 9,
**dadurch gekennzeichnet,**
**dass** ein dem Benutzer angewandter Arm (8) länger als die übrigen Arme (8) ausgebildet ist.

11. Strahlenschutzvorrichtung nach Anspruch 9 oder 10,
**dadurch gekennzeichnet,**
**dass** an den Armen (8) endständige bremsbare Rollen (9) angeordnet sind.

12. Strahlenschutzvorrichtung nach Anspruch 1,
**dadurch gekennzeichnet,**
**dass** eine Höhenverstellung (10) zumindest eine Gasdruckfeder (11) umfasst.

13. Strahlenschutzvorrichtung nach Anspruch 12,
**dadurch gekennzeichnet,**
**dass** die zumindest eine Gasdruckfeder (11) in der zumindest einer Leiste (6) angeordnet ist.

14. Strahlenschutzvorrichtung nach Anspruch 1,
**dadurch gekennzeichnet,**
**dass** die Höhenverstellung (10) in Form einer mechanischen Höhenverstellung (10), einer hydraulischen Höhenverstellung (10) oder einer elektrischen Höhenverstellung (10) ausgebildet ist.

15. Strahlenschutzvorrichtung nach einem der Ansprüche 1 bis 14,
**dadurch gekennzeichnet,**
**dass** die Strahlenschutzscheibe (2) Ausnehmungen (5) aufweist.

16. Strahlenschutzvorrichtung nach Anspruch 15,
**dadurch gekennzeichnet,**
**dass** die Ausnehmungen (5) der Strahlenschutzscheibe (2) asymmetrisch sind.

17. Strahlenschutzvorrichtung nach Anspruch 15 oder 16,
**dadurch gekennzeichnet,**
**dass** die Ausnehmungen (5) durch Bleilappen (15) abdeckbar sind.

18. Strahlenschutzvorrichtung nach Anspruch 17,
**dadurch gekennzeichnet,**
**dass** die Bleilappen (15) mittels Leisten (13), Druckknöpfen (14) oder Klettbändern an der Strahlenschutzscheibe (2) fixiert sind.

19. Strahlenschutzvorrichtung nach einem der Ansprüche 1 bis 18,
**dadurch gekennzeichnet,**
**dass** die Strahlenschutzscheibe (2) an einem unteren Ende eine Bleischürze (12) aufweist.

20. Strahlenschutzvorrichtung nach Anspruch 19,
**dadurch gekennzeichnet,**
**dass** die Bleischürze (12) mittels Leisten (13), Druckknöpfen (14) oder Klettbändern an der Strahlenschutzscheibe (2) fixiert ist.

21. Strahlenschutzvorrichtung nach Anspruch 19 oder 20,
**dadurch gekennzeichnet,**
**dass** die Bleischürze (12) aus mehreren, einander überlappenden Schürzenteilen (18) besteht.

22. Strahlenschutzvorrichtung nach einem der Ansprüche 1 bis 21,
**dadurch gekennzeichnet,**
**dass** die Strahlenschutzvorrichtung (1) Handgriffe (16) aufweist.

23. Strahlenschutzvorrichtung nach Anspruch 22,
**dadurch gekennzeichnet,**
**dass** die Handgriffe (16) seitlich in Ellenbogenhöhe an der Strahlenschutzscheibe (2) angebracht sind.

24. Strahlenschutzvorrichtung nach Anspruch 22 oder 23,
**dadurch gekennzeichnet,**
**dass** die Handgriffe (16) herunterklappbar sind.

25. Strahlenschutzvorrichtung nach einem der Ansprüche 1 bis 24,
**dadurch gekennzeichnet,**
**dass** die Strahlenschutzscheibe (2) in einem Sockel (17) angeordnet ist.

26. Strahlenschutzvorrichtung nach Anspruch 25,
**dadurch gekennzeichnet,**
**dass** der Sockel (17) mit dem Fuß (7) verbunden ist.

27. Strahlenschutzvorrichtung nach Anspruch 25 oder 26,
**dadurch gekennzeichnet,**
**dass** der Sockel (17) unter dem gleichen Krümmungsradius wie die Strahlenschutzscheibe (2) gekrümmt ist.

28. Strahlenschutzvorrichtung nach einem der Ansprüche 25 bis 27,
**dadurch gekennzeichnet,**
**dass** der Fuß (7) unter dem gleichen Krümmungsradius wie der Sockel (17) gekrümmt ist.

29. Strahlenschutzvorrichtung nach einem der Ansprüche 25 bis 28,
**dadurch gekennzeichnet,**
**dass** der Sockel (17) Strahlenschutzmaterial enthält.

30. Strahlenschutzvorrichtung nach einem der Ansprüche 25 bis 29,
**dadurch gekennzeichnet,**
**dass** in den Sockel (17) eine Höhenverstellung (10) integriert ist.

31. Strahlenschutzvorrichtung nach einem der Ansprüche 1 bis 30,
**dadurch gekennzeichnet,**
**dass** die Strahlenschutzscheibe (2) aus Bleiacryl besteht.

## Claims

1. Radiation protection device (1) for screening from radiation which is radiated from a radiation source, in particular an X-ray source, the radiation protection device (1) including a transparent radiation protection plate (2) which absorbs the radiation, and is fixed on a stand device (3),
**characterized in that**
the radiation protection plate (2) is in curved form, and can be guided close to the body when an operator's body is in a standing position,
the radiation protection plate (2) being fixed to the stand device (3) at an adjustable height, so that the lower extremities of the operator are screened from the radiation at all times.

2. Radiation protection device according to Claim 1,
**characterized in that**
the stand device (3) includes at least one batten (6).

3. Radiation protection device according to Claim 2,
**characterized in that**
the radiation protection plate (2) is fixed to the at least one batten (6).

4. Radiation protection device according to Claim 3,
**characterized in that**
the fixing is by screws (4), bolts or similar fixing devices.

5. Radiation protection device according to one of Claims 2 to 4,
**characterized in that**
the at least one batten (6) is fixed to the stand device (3) and the radiation protection plate (2), and extends parallel to the latter.

6. Radiation protection device according to Claim 1,
**characterized in that**
two battens (6) are provided.

7. Radiation protection device according to Claim 6,
**characterized in that**
the two battens (6) extend along outer edges (17) of the radiation protection plate (2).

8. Radiation protection device according to one of Claims 1 to 7,
**characterized in that**
the stand device (3) has a foot (7) in the form of a double cross.

9. Radiation protection device according to Claim 8,
**characterized in that**
the foot (7) has four arms (8).

10. Radiation protection device according to Claim 9,
**characterized in that**
an arm (8) which is applied to the user is in longer form than the other arms (8).

11. Radiation protection device according to Claim 9 or 10,
**characterized in that**
rollers (9), which can be braked, are arranged on the ends of the arms (8).

12. Radiation protection device according to Claim 1,
**characterized in that**
a height adjuster (10) includes at least one gas pressure spring (11).

13. Radiation protection device according to Claim 12,
**characterized in that**
the at least one gas pressure spring (11) is arranged in the at least one batten (6).

14. Radiation protection device according to Claim 1,
**characterized in that**
the height adjuster (10) is in the form of a mechanical height adjuster (10), a hydraulic height adjuster (10) or an electrical height adjuster (10).

15. Radiation protection device according to one of Claims 1 to 14,
**characterized in that**
the radiation protection plate (2) has recesses (5).

16. Radiation protection device according to Claim 15,
**characterized in that**
the recesses (5) of the radiation protection plate (2) are asymmetrical.

17. Radiation protection device according to Claim 15 or 16,
**characterized in that**
the recesses (5) can be covered by lead tabs (15).

18. Radiation protection device according to Claim 17,
**characterized in that**
the lead tabs (15) are fixed to the radiation protection plate (2) by means of battens (13), snap fasteners (14) or Velcro® strips.

19. Radiation protection device according to one of Claims 1 to 18,
**characterized in that**
the radiation protection plate (2) has a lead apron (12) at a lower end.

20. Radiation protection device according to Claim 19,
**characterized in that**
the lead apron (12) is fixed to the radiation protection plate (2) by means of battens (13), snap fasteners (14) or Velcro® strips.

21. Radiation protection device according to Claim 19 or 20,
**characterized in that**
the lead apron (12) consists of multiple overlapping apron parts (18).

22. Radiation protection device according to one of Claims 1 to 21,
**characterized in that**
the radiation protection device (1) has handles (16).

23. Radiation protection device according to Claim 22,
**characterized in that**
the handles (16) are fitted laterally at elbow height on the radiation protection plate (2).

24. Radiation protection device according to Claim 22 or 23,
**characterized in that**
the handles (16) can be folded down.

25. Radiation protection device according to one of Claims 1 to 24,
**characterized in that**
the radiation protection plate (2) is arranged in a base (17).

26. Radiation protection device according to Claim 25,
**characterized in that**
the base (17) is connected to the foot (7).

27. Radiation protection device according to Claim 25 or 26,
**characterized in that**
the base (17) is curved at the same radius of curvature as the radiation protection plate (2).

28. Radiation protection device according to one of Claims 25 to 27,
**characterized in that**
the foot (7) is curved at the same radius of curvature as the base (17).

29. Radiation protection device according to one of Claims 25 to 28,
**characterized in that**
the base (17) contains radiation protection material.

30. Radiation protection device according to one of Claims 25 to 29,
**characterized in that**
a height adjuster (10) is integrated in the base (17).

31. Radiation protection device according to one of Claims 1 to 30,
**characterized in that**
the radiation protection plate (2) consists of lead acrylic.

## Revendications

1. Dispositif de protection (1) contre les radiations ou rayonnements destiné à former écran à l'encontre de radiations rayonnées par une source de radiations, notamment une source de rayons X,
le dispositif de protection (1) contre les radiations comprenant une vitre de protection (2) contre les radiations, qui est transparente, absorbe les radiations et est fixée à un dispositif de support vertical (3),
**caractérisé**
**en ce que** la vitre de protection (2) contre les radiations est réalisée bombée et peut être amenée proche du corps d'une personne de service dans une position corporelle debout,
la vitre de protection (2) contre les radiations étant fixée de manière réglable en hauteur sur le dispositif de support vertical (3), de sorte que les extrémités inférieures de la personne de service sont à tout moment masquées à l'encontre des radiations.

2. Dispositif de protection contre les radiations selon la revendication 1,
**caractérisé en ce que** le dispositif de support vertical (3) comprend au moins un montant en forme de barre (6).

3. Dispositif de protection contre les radiations selon la revendication 2,
**caractérisé en ce que** la vitre de protection (2) contre les radiations est fixée sur ledit au moins un montant en forme de barre (6).

4. Dispositif de protection contre les radiations selon la revendication 3,
**caractérisé en ce que** la fixation est effectuée au moyen de vis (4), de boulons ou de dispositifs de fixation similaires.

5. Dispositif de protection contre les radiations selon l'une des revendications 2 à 4,
**caractérisé en ce que** ledit au moins un montant en forme de barre (6) est fixé au dispositif de support vertical (3) et à la vitre de protection (2) contre les radiations.

6. Dispositif de protection contre les radiations selon la revendication 1,
**caractérisé en ce que** sont prévus deux montants en forme de barre (6).

7. Dispositif de protection contre les radiations selon la revendication 6,
**caractérisé en ce que** les deux montants en forme de barre (6) s'étendent le long de bords extérieurs (17) de la vitre de protection (2) contre les radiations.

8. Dispositif de protection contre les radiations selon l'une des revendications 1 à 7,
**caractérisé en ce que** le dispositif de support vertical (3) présente un pied (7) en forme de croix double.

9. Dispositif de protection contre les radiations selon la revendication 8,
**caractérisé en ce que** le pied (7) comporte quatre bras (8).

10. Dispositif de protection contre les radiations selon la revendication 9,
**caractérisé en ce qu'**un bras (8) dirigé vers l'utilisateur est réalisé plus long que les autres bras (8) restants.

11. Dispositif de protection contre les radiations selon la revendication 9 ou la revendication 10,
**caractérisé en ce que** sur les bras (8), à l'extrémité de ceux-ci, sont agencées des roulettes (9) pouvant être freinées.

12. Dispositif de protection contre les radiations selon la revendication 1,
**caractérisé en ce qu'**un système de réglage en hauteur (10) comprend au moins un ressort de compression à gaz (11).

13. Dispositif de protection contre les radiations selon la revendication 12,
**caractérisé en ce que** ledit au moins un ressort de compression à gaz (11) est agencé dans ledit au moins un montant en forme de barre (6).

14. Dispositif de protection contre les radiations salon la revendication 1,
**caractérisé en ce que** le système de réglage en hauteur (10) est réalisé sous la forme d'un système de réglage en hauteur (10) mécanique, d'un système de réglage en hauteur (10) hydraulique ou d'un système de réglage en hauteur (10) électrique.

15. Dispositif de protection contre les radiations selon l'une des revendications 1 à 14,
**caractérisé en ce que** la vitre de protection (2) contre les radiations présente des évidements ou encoches (5).

16. Dispositif de protection contre les radiations selon la revendication 15,
**caractérisé en ce que** les encoches (5) de la vitre de protection (2) contre les radiations, sont asymétriques.

17. Dispositif de protection contre les radiations selon la revendication 15 ou la revendication 16,
**caractérisé en ce que** les encoches (5) peuvent être recouvertes ou masquées par des bavettes de plomb (15).

18. Dispositif de protection contre les radiations selon la revendication 17,
**caractérisé en ce que** les bavettes de plomb (15) sont fixées à la vitre de protection (2) contre les radiations, au moyen de barrettes (13), de boutons pression (14) ou de bandes auto-agrippantes.

19. Dispositif de protection contre les radiations selon l'une des revendications 1 à 18,
**caractérisé en ce qu'**au niveau d'une extrémité inférieure, la vitre de protection (2) contre les radiations comporte un tablier de plomb (12).

20. Dispositif de protection contre les radiations selon la revendication 19,
**caractérisé en ce que** le tablier de plomb (12) est fixé à la vitre de protection (2) contre les radiations, au moyen de barrettes (13), de boutons pression (14) ou de bandes auto-agrippantes.

21. Dispositif de protection contre les radiations selon la revendication 19 ou la revendication 20,
**caractérisé en ce que** le tablier de plomb (12) est constitué de plusieurs parties de tablier (18) se chevauchant mutuellement.

22. Dispositif de protection contre les radiations selon l'une des revendications 1 à 21,
**caractérisé en ce que** le dispositif de protection (1) contre les radiations comporte des poignées (16).

23. Dispositif de protection contre les radiations selon la revendication 22,
**caractérisé en ce que** les poignées (16) sont placées latéralement sur la vitre de protection (2) contre les radiations, à hauteur de coude.

24. Dispositif de protection contre les radiations selon la revendication 22 ou la revendication 23,
**caractérisé en ce que** les poignées (16) sont rabattables.

25. Dispositif de protection contre les radiations selon l'une des revendications 1 à 24,
**caractérisé en ce que** la vitre de protection (2) contre les radiations est agencée dans une embase (17).

26. Dispositif de protection contre les radiations selon la revendication 25,
**caractérisé en ce que** l'embase (17) est reliée au pied (7).

27. Dispositif de protection contre les radiations selon la revendication 25 ou la revendication 26,
**caractérisé en ce que** l'embace (17) est courbée selon le même rayon de courbure que la vitre de protection (2) contre les radiations.

28. Dispositif de protection contre les radiations selon l'une des revendications 25 à 27,
**caractérisé en ce que** le pied (7) est courbé selon le même rayon de courbure que l'embase (17).

29. Dispositif de protection contre les radiations selon l'une des revendications 25 à 28,
**caractérisé en ce que** l'embase (17) renferme du matériau de protection contre les radiations.

30. Dispositif de protection contre les radiations selon l'une des revendications 25 à 29,
**caractérisé en ce qu'**un système de réglage en hauteur (10) est intégré à l'embase (17).

31. Dispositif de protection contre les radiations selon l'une des revendications 1 à 30,
**caractérisé en ce que** la vitre de protection (2) contre les radiations est réalisée en verre acrylique au plomb.
